(19) Europäisches Patentamt

European Patent Office

Office européen des brevets

(11) Veröffentlichungsnummer: **0 421 376 B1**

(12) **EUROPÄISCHE PATENTSCHRIFT**

(45) Veröffentlichungstag der Patentschrift: **19.04.95**

(21) Anmeldenummer: **90118938.1**

(22) Anmeldetag: **04.10.90**

(51) Int. Cl.⁶: **C12N 9/00**, C12N 15/11, C12N 5/10, A01H 5/00, A01N 57/16

(54) **Multifunktionelle RNA mit Selbstprozessierungsaktivität, ihre Herstellung und Verwendung.**

(30) Priorität: **06.10.89 DE 3933384**

(43) Veröffentlichungstag der Anmeldung:
**10.04.91 Patentblatt 91/15**

(45) Bekanntmachung des Hinweises auf die Patenterteilung:
**19.04.95 Patentblatt 95/16**

(84) Benannte Vertragsstaaten:
**AT BE CH DE DK ES FR GB IT LI LU NL**

(56) Entgegenhaltungen:
**EP-A- 0 321 201**

**BIOTECHNOLOGY, Band 6, Nr. 5, Mai 1988, Seiten 549-557, New York, NY, US; M.CUOZZO et al.: "Viral protection in transgenic tobacco plants expressing thecucumber mosaic virus coat protein or its antisense RNA"**

**PLANT MOLECULAR BIOLOGY, Band 11, 1988, Seiten 463-471, Kluwer AcademicPublishers, Dordrecht, NL; M.A. REZAIAN et al.: "Anti-sense RNAs of cucumbermosaic virus in transgenic plants assessed for control of**

**the virus"**

(73) Patentinhaber: **Hoechst Schering AgrEvo GmbH**
**Gerichtstrasse 27**
**D-13342 Berlin (DE)**

(72) Erfinder: **Müllner, Hubert, Dr.**
**Staufenstrasse 1**
**W-6233 Kelkheim (Taunus) (DE)**
Erfinder: **Uhlmann, Eugen, Dr.**
**Zum Talblick 31**
**W-6246 Glashütten/Taunus (DE)**
Erfinder: **Eckes, Peter, Dr.**
**Am Flachsland 18**
**W-6233 Kelkheim (Taunus) (DE)**
Erfinder: **Schneider, Rudolf, Dr.**
**Feldbergstrasse 98**
**W-6233 Kelkheim (Taunus) (DE)**
Erfinder: **Uijtewaal, Bernardus,Dr.**
**In de Neerakker 68**
**NL-6093 JG Heythuysen (NL)**

**Beschreibung**

RNA-Moleküle können unter geeigneten Voraussetzungen ohne Beteiligung von Proteinen an anderen RNA-Molekülen Reaktionen katalysieren oder autokatalytisch Fragmente aus eigenen Molekülen abspalten. So wird vom 3′-Ende der 23S rRNA von Tetrahymena thermophila autokatalytisch ein Intron mit 413 Nukleotiden entfernt und in eine zirkuläre Form übergeführt. Dies geschieht durch eine Reihe von Phosphoestertransfer-Reaktionen mit Beteiligung von Guanosin-Kofaktoren (Cech, T.R., Nature 30, 578-583 (1983)). Je nach RNA Substrat oder den gewählten Reaktionsbedingungen kann das Intron als spezifische Ribonuclease, terminale Transferase, Phosphotransferase oder saure Phosphatase funktionieren. Dabei kann ein RNA-Molekül einige Umsetzungen vornehmen, ohne selbst verändert zu werden und verhält sich in dieser Hinsicht wie ein Enzym. Für RNA-Moleküle mit diesen Eigenschaften wurde deshalb der Begriff Ribozym geprägt.

Ähnliche Reaktionen ohne Beteiligung von Proteinen konnten auch für einige viroide RNAs und Satelliten-RNAs gezeigt werden. So scheint für Avocado Sunblotch Viroid (ASBV) (Hutchins, C.J. et al. Nucleic Acids Res. 14, 3627-3640 (1986)), Satelliten-RNA von Tobacco Ringspot Virus (sTobRV) (Prody, G.A. et a.l, Science 231, 1577-1580 (1986)) und Satelliten-RNA von Luzerne Transient Streak Virus (sLTSV) (Forster A.C. et al., Cell 49, 211-220 (1987)) eine Selbst-Prozessierung eine essentielle Reaktion für die Vermehrung zu sein. Während der Replikation dieser RNAs werden vermutlich zirkuläre Formen gebildet, die als "Templates" zur Synthese von RNAs mit Überlänge führen. Diese Transkripte werden durch die selbstkatalysierten, endonucleolytischen Reaktionen auf die richtige Genomlänge zurechtgeschnitten.

Die Strukturen der RNAs, die diese vermutlich für die Reduktion einnehmen, wurden als "hammerheads" beschrieben (Forster A.C. et al., Cell 49, 211-220 (1987); Haseloff, J. et al., Nature 334, 585-591 (1988)).

Die Schnittstellen für diese RNA-Enzyme sind spezifisch und müssen bestimmte strukturelle Voraussetzungen aufweisen, damit eine Prozessierung erfolgen kann.

Es wurde nun gefunden, daß Wirtszellen beliebiger Organismen mit Vektoren, die DNA kodierend für Ribozym-RNA verknüpft mit "antisense"-RNA enthalten, transformiert werden können, so daß die genannte DNA exprimiert wird.

Es ist bekannt, daß "antisense"-RNA die Gen-Expression in einer Reihe von prokaryotischen und eukaryotischen Zellen, unter anderem auch in Pflanzenzellen, inhibiert (Green, P.J. et al., Ann. Rev. Biochem. 55, 569 (1986)). Der Hemmungs-Mechanismus ist noch weitgehend ungeklärt. Man nimmt an, daß sich in eukaryotischen Systemen doppelsträngige RNA bildet, die den Transport der m-RNA zum Cytoplasma behindert.

Rezaian, M. et al. (Plant Mol. Biol. 11, 463 (1988)) untersuchten beispielsweise die Möglichkeit der Verwendung von "antisense"-RNA als antivirales Agens gegen Cucumber Mosaic Virus (CMV). Die Autoren beobachteten jedoch, daß die antivirale Effektivität der antisense-RNA unbefriedigend war.

Die Verknüpfung der entsprechenden Ribozym-RNA mit der entsprechenden antisense-RNA über einen Spacer bewirkt nun jedoch z.B. eine effektivere Virusabwehr als Rezaian sie zeigen konnte. Eine derartige Verknüpfung der RNA-Moleküle bewirkt also nicht nur bezogen auf die Aktivität als antivirales Agens in Pflanzen, sondern allgemein eine verstärkte, gegen ein Substrat gerichtete Aktivität in transformierten Organismen.

1. Gen, das für eine multifunktionelle RNA kodiert, die in 5'-3'-Richtung der Transcription umfaßt:
   a, Ribozym-RNA
   b, Spacer
   c, (Antisense-RNA)$_n$-,
   d, Spacer und
   e, Ribozym-RNA
   wobei
   - n eine Zahl von 0 bis 10 ist,
   - als Spacer eine Kette von 20 bis 25 Nukleotiden eingeschoben wird, die von dem Ribozym geschnitten werden kann und

- die Ribozyme die folgende Struktur aufweisen:

```
5-NNNNNNNNNNNNGUC NNNNNNNNNNNNNN-3
3-KKKKKKKCA  KKKKKKK-5     ↘
           A      C
           A       U
           G        G
           C   A   A
            G  G  G
              V V
              V V
             Vₗ Vₗ
              V V
```

worin

N       Nukleotide der Substrat-RNA, A, C, G oder U,

K       komplementäre Nukleotide zu N im Ribozym,

V       variable Nukleotide A, C, G oder U im Ribozym

$V_L$   0-550 variable Nukleotide A, C, G oder U im Loop des Ribozyms bedeutet.

2. Organismen, die das unter 1. charakterisierte Gen oder die entsprechende RNA-Sequenz enthalten.

3. Die Verwendung von Ribozym-RNA über einen Spacer verknüpft mit "antisense"-RNA, als gegen ein Substrat gerichtetes Agens in Organismen.

Im folgenden wird die Erfindung detailliert beschrieben, insbesondere in ihren bevorzugten Ausführungsformen. Ferner wird die Erfindung in den Ansprüchen definiert.

Die erfindungsgemäße multifunktionelle RNA ist im wesentlichen so zusammengesetzt, daß sich die Ribozyme jeweils am 3'- bzw. 5'-Ende des RNA-Moleküls befinden. Über sogenannte "Spacer" sind "antisense"-RNA Einheiten dazwischengeschaltet, die, wenn es sich um mehrere Einheiten handelt, ebenfalls über "Spacer" miteinander verbunden sind. Bevorzugte Ausführungsformen des multifunktionellen RNA-Moleküls können in einem Schema dargestellt wie folgt aussehen:

5'-Ribozym RNA-Spacer-(antisense RNA)ₙ-Spacer-Ribozym RNA-3' wobei n eine Zahl von 1 bis 10, bevorzugt 1 bis 5, insbesondere 1 bis 3 ist. Als Spacer wird eine Kette von 20 bis 25 Nukleotiden eingeschoben, die im ganzen oder in einem mindestens 8, bevorzugt 10-20 Nukleotide zählenden Teilbereich zur Sequenz des Ribozyms komplementär ist. Die Ribozym-Sequenz der RNA kann sich so an den Spacer anlagern und diesen unmittelbar hinter eine Erkennungssequenz schneiden. Wenn die Zahl n größer 1 ist, wird zwischen die jeweiligen antisense RNA-Moleküle ebenfalls ein derartiger Spacer mit Ribozym Schnittstelle geschaltet. Als Ribozym-Schnittstelle wird bevorzugt eine GUC-Schnittstelle eingebaut. Die "antisense"-RNA kann gegen Substrate, wie beispielsweise RNA codierend für selektierbare Markergene (Antibiotika-Resistenzen) oder RNA codierend für beliebige Zellfunktionen, wie z.B. Dihydrofolatreduktase, Thymidinkinase, die Reifungsenzyme Polygalekturonase, Pektinesterase etc., Proteine verantwortlich für Differenzierung und Entwicklung oder Hormonrezeptoren gerichtet sein. Insbesondere pflanzenschädigende Virusarten können vorteilhaft mit dem erfindungsgemäßen Ribozym-"antisense" System bekämpft werden. Dazu wird beispielsweise wie im folgenden beschrieben vorgegangen. In analoger Arbeitsweise wird auch die Ribozym-"antisense"-RNA synthetisiert, die gegen andere Substrate gerichtet ist.

Die für die multifunktionelle RNA kodierenden DNA-Oligonukleotide können synthetisch hergestellt werden. Die Oligonukleotide für die "antisense"-RNA können auf Basis der viralen DNA- bzw. RNA-Sequenzen synthetisiert werden. Dabei kann grundsätzlich jedes pflanzenschädigende Virus herangezogen werden. Bevorzugte Virusarten sind pathogene RNA-Viren, insbesondere Cucumber Mosaik Virus, Alfalfa Mosaik Virus, Brom Mosaik Virus, Tabak Mosaik Virus, Kartoffel-Virus X oder Y, Tomaten Ringspot Virus, Tomaten Aspermy Virus oder Tabak-Rattle Virus.

Bevorzugt werden als "Vorlage" für die Synthese der Ribozym-RNA kodierenden Oligonukleotide mindestens 10 aufeinanderfolgende Nukleotide, insbesondere 14 bis 20 Nukleotide, vorteilhaft aus der Mitte der RNA-Sequenz des entsprechenden Virus verwendet. Bei dieser RNA-Sequenz kann es sich sowohl um das Genom von RNA-Viren handeln als auch um eine RNA-Sequenz, die von der DNA-Sequenz eines DNA-Virus abgeleitet wurde. Es ist insbesondere vorteilhaft, jeweils von den 3 RNA-Sequenzen, RNA1, RNA2 und RNA3 des Cucumber Mosaic Virus oder Teilen davon auszugehen, entsprechend der Sequenzen aus den Publikationen von Rezaian M. et al., Eur. J. Biochem. 150, 331-339 (1985); Eur. J. Biochem. 143, 277-284 (1984) und Gould I. et al., Eur. J. Biochem. 126, 217-226 (1982).

Die Ribozym kodierenden Oligonukleotide werden so synthetisiert, daß die jeweils aus mindestens 5 Nukleotiden, vorteilhaft 7 bis 10 Nukleotiden bestehenden Anfangs- bzw. Endsequenzen komplementär zu der RNA des zu hemmenden Virus sind. Die dazwischenliegende Sequenz besteht zum Teil aus spezifischen, für die Funktionalität des Ribozyms vorgegebenen Nukleotiden und zum Teil aus variablen Nukleotiden. Das mit Substrat-RNA hybridisierte Ribozym kann im Schema wie folgt aussehen:

```
5-NNNNNNNNNNNNNGUC NNNNNNNNNNNNNN-3   ←——— Substrat-RNA
   3-KKKKKKKKCA  KKKKKKK-5         ⎫
            A  C                   ⎪
            A   U                  ⎪
            G    G                 ⎪
            C G A  A               ⎬  Ribozym
              G  G V               ⎪
            V V                    ⎪
            V V                    ⎪
            V V                    ⎪
            V  V                   ⎭
```

wobei

N Nukleotide der Substrat-RNA, A, C, G oder U

K komplementäre Nukleotide zu N im Ribozym

V variable Nukleotide A, C, G oder U im Ribozym und

$V_L$ variable Nukleotide A, C, G oder U im Loop des Ribozyms bedeutet.

Die Nukleotidanzahl von $V_L$ kann 0-550 betragen.

Bei der Herstellung der antisense-RNA wird entsprechend vorgegangen, nur werden die Oligonukleotide so synthetisiert, daß sie für eine RNA in entsprechender gegensinniger Orientierung kodieren.

Zur Oligonukleotid-Synthese für die Ribozym RNA kann aber auch von einem ganz beliebigen Substrat ausgegangen werden. Zu beachten ist eigentlich nur, daß der Spacer ein Substrat für das Ribozym darstellt und mit den entsprechenden Ribozymschnittstellen versehen ist. Die Spacer zur Verknüpfung der Ribozym und antisense-Moleküle werden so konstruiert, daß die entsprechend exprimierte RNA im ganzen oder in Teilen komplementär zum Ribozym ist, wobei sich vorteilhaft die komplementären Nukleotide um eine GUC-Schnittstelle für das Ribozym gruppieren.

Die konstruierten Oligonukleotide werden mit einem entsprechenden Linker versehen. Derartige Linker besitzen beispielsweise Schnittstellen von EcoRI, SalI, BamHI, HindIII, EcoRV, SmaI, XhoI, KpnI, bevorzugt XbaI bzw. PstI.

Die zusammengesetzten Oligonukleotide werden mit Hilfe der Vektoren pUC19, pUC18 oder pBluescript (Stratagene, Heidelberg, Product Information) kloniert, und sequenziert.

Das bestätigte Oligonukleotid wird in einen intermediären Vektor mit Pflanzenpromotor kloniert. Derartige Vektoren sind beispeilsweise die Plasmide pPCV701 (Velten J. et al. EMBO J. 3, 2723-2730 (1984)), pNCN (Fromm M. et al. PNAS 82, 5824-5826 (1985)) oder pNOS (An G. et al., EMBO J. 4, 277-276 (1985)). Bevorzugt wird der Vektor pDH51 (Pietrzak, M. et al., NAR 14, 5857, (1986)) mit einem 35S-Promotor verwendet.

Nach anschließender Transformation von E. coli, wie z.B. E. coli MC 1061, DH1, DK1, GM48 oder XL-1, werden positive Klone nach an sich bekannten Methoden (Maniatis et al., Lab. Manual), wie Plasmidminipräparation und Spaltung mit einem entsprechenden Restriktionsenzym, identifiziert.

Diese positiven Klone werden dann in einen binären Pflanzenvektor subkloniert. Als Pflanzenvektoren können pGV3850 (Zambrysky, P. et al, EMBO J. 2, 2143-2150 (1983)) oder pOCA18 (Olszewski, N., NAR 16, 10765-10782, (1988)) eingesetzt werden. Vorteilhaft wird mit pOCA18 gearbeitet.

Die erhaltenen binären Pflanzenvektoren, die einen Pflanzenpromotor mit dem angehängten DNA-Fragment, das wie oben angegeben konstruiert ist, in der T-DNA enthalten, werden verwendet, um Pflanzen zu transformieren. Dies kann durch Techniken wie Elektroporation oder Mikroinjektion erfolgen.

Bevorzugt wird die Kokultivierung von Protoplasten oder die Transformation von Blattstückchen mit Agrobakterien angewandt. Dazu wird das Pflanzenvektorkonstrukt durch Transformation mit gereinigter DNA oder, vermittelt über einen Helferstamm wie E. coli SM10 (Simon R. et al., Biotechnology 1, 784-791 (1983)), in Agrobakterium tumefaciens wie A 282 mit einem Ti Plasmid über ein "triparental mating" transferiert. Direkte Transformation und triparental mating wurden, wie in, "Plant Molecular Biology Manual" (Kluwer Academic Publishers, Dardrecht (1988)) beschrieben, durchgeführt.

Es können grundsätzlich alle Pflanzen mit den die erfindungsgemäße, konstruierte DNA tragenden binären Pflanzenvektoren transformiert werden. Bevorzugt sind dikotyledone Pflanzen, insbesondere Nutzpflanzen, die beispielsweise Stärke, Kohlenhydrate, Eiweiße oder Fette in nutzbaren Mengen in ihren Organen produzieren oder speichern oder die Obst und Gemüse produzieren oder die Gewürze, Fasern und technisch verwertbare Produkte oder Arzneimittel, Farbstoffe oder Wachse liefern ebenso wie Futterpflanzen.

Als Beispiel sollen Tomate, Erdbeere, Avocado, sowie Pflanzen, die tropische Früchte tragen, z.B. Papaya, Mango, aber auch Birne, Apfel, Nektarine, Aprikose oder Pfirsich genannt werden. Ferner sollen als zu transformierende Pflanzen beispielhaft alle Getreidearten, Raps, Kartoffeln, Sojabohne, Baumwolle, Mais, Zuckerrübe oder Sonnenblume, aufgeführt werden. Die transformierten Zellen werden mit Hilfe eines Selektionsmediums selektiert, zu einem Kallus herangezüchtet und auf einem entsprechenden Medium zur Pflanze regeneriert (Shain et al, Theor. appl. Genet. 72, 770-770 (1986); Masson, J. et al., Plant Science 53, 167-176 (1987), Zhan et al., Plant Mol. Biol. 11, 551-559 (1988); McGranaham et al, Bio/Technology 6, 800-804 (1988) Novrate et al., Bio/Technology 7, 154-159 (1989).

Die resultierende Pflanze wird durch die Transformation insofern verändert, als in den Zellen die mit Hilfe der konstruierten Oligonukleotide exprimierte RNA durch die Ribozymaktivität an GUC-Schnittstellen aufgespalten wird, antisense RNA dadurch freigesetzt wird, wobei Ribozym RNA zusammen mit antisense RNA gegenüber Virus-DNA bzw. -RNA aktiv werden kann.

Die folgenden Beispiele dienen der weiteren Erläuterung der Erfindung.

Beispiele

Prozentangaben beziehen sich auf das Gewicht, soweit nichts anderes angegeben.

**1. Synthese der DNA zur Expression der multifunktionellen RNA**

Die Synthese der DNA-Sequenz der Tabelle 1 zur Expression der multifunktionellen RNA erfolgte wie nachfolgend beschrieben:

a) Ribozym I [1 - 46]: die CMV homologen Bereiche basieren auf der publizierten RNA 1 Sequenz (Rezaian M. et al., Eur. J. Biochem. 150, 331-339/1985) von Position 3248 bis 3264. Die konstanten Regionen für das Ribozym sind aus Tabelle 1 ersichtlich und wurden aus der Publikation von Haseloff, J. et al., Nature 334, 585-591 (1988) abgeleitet.

b) Spacer [47 - 70]: auf Basis der publizierten RNA 2-Sequenz (Rezaian, M. et al., Eur. J. Biochem. 143, 277-284 (1984) von Position 2853 bis 2870.

c) Antisense 1 [71 - 243]: auf Basis der publizierten RNA 4-Sequenz (Gould I. et al., Eur. J. Biochem. 143, 217-226 (1982) von Position 2054 bis 2193.

d) Spacer [219 - 243]: wie für Spacer [47 -70] unter b.

e) Antisense 2 [244 - 313]: auf Basis der publizierten RNA 2-Sequenz (s.o.) von Position 71 bis 134.

f) Spacer [314 - 338]: wie für Spacer [47 - 70] unter b.

g) Antisense 3 [339 - 405]: auf Basis der publizierten RNA 4-Sequenz (s.o.) von Position 1200 bis 1261.

h) Spacer [406 - 429]: Auf Basis der publizierten RNA 1-Sequenz (s.o.) von Position 3248 bis 3264.

i) Ribozym [430 - 480]: für die CMV homologen Bereiche auf Basis der RNA 2-Sequenz (s.o.) von Position 2853 bis 2870; für die konstanten Regionen nach Tab. 1 siehe Publikation unter a).

Die eckigen Klammern beziehen sich auf die Positionen der beigefügten DNA-Sequenz zur Expression der multifunktionellen RNA (Tab. 1).

Mit Hilfe der Phosporamidit-Methode wurden die in Tabelle 1 aufgeführten Oligonukleotide mit einem Synthesizer synthetisiert. Die Fragmente sind aus der Sequenz ersichtlich. Das erste reicht vom XmaI Linker am Anfang bis zur KpnI-Stelle, das zweite von KpnI bis BamHI, das dritte von BamHI bis SacI und das vierte von SacI bis zum SmaI Übergang am Ende. Synthetisiert wurden jeweils Strang und Gegenstrang, die vor der Klonierung in gleichen molaren Mengen zusammengegeben wurden.

## 2. Klonierung in pBluescript SK+ und Sequenzierung

Das Plasmid pBluescript SK+ (Stratagene, Product Information) wurde mit den jeweiligen Enzymen (XmaI/KpnI, KpnI/BamHI, BamHI/SacI, SacI/SmaI) geöffnet und nach Behandlung mit "calf intestinal phosphatase" (CIP) mit einem fünffachen Überschuß der doppelsträngigen phosphorylierten Oligonukleotide ligiert. Positive Klone konnten in dem Stamm XL-Blue (Stratagene) nach Induktion mit Isopropylthiogalactosid (IPTG, Fa. Boehringer Mannheim) auf LB-Platten mit 5-Brom-4-chlor-3-indolyl-$\beta$-D-galaktosid (X-gal) (Fa. Boehringer, Mannheim) als weiße Kolonien identifiziert werden.

Jeweils 5 der Kolonien wurden isoliert und nach der Dideoxymethode sequenziert (Boehringer, Sequencing Kit). Aus einer Kolonie mit der erwarteten Sequenz wurde das Oligonukleotid nach Spaltung mit den betreffenden Enzymen aus einem hochgereinigten (Low Melt) Agarose Gel (Fa. Gibco, Brueggenstein, BR Deutschland) isoliert.

Die isolierten Oligonukleotide wurden ligiert und auf einem hochgereinigten (Low Melt) Agarose Gel von nicht ligierten abgetrennt. Die erwartete 0,5 kb-Bande wurde ausgeschnitten und nach dem Auffüllen der überhängenden XmaI-Stelle in die SmaI-Erkennungssequenz von pBluescript SK+ eingesetzt.

Das Foto zeigt 6 Kolonien einer Plasmidpräparation aus XL-1 Blue Zellen, die mit dem Vektor pBluescript, in dem das vollständige Fragment eingebaut war, transformiert wurden. Die Plasmide wurden vor dem Auftragen auf das Gel SmaI geschnitten. In den Kolonien 1-5 ist ein Fragment der erwartenden Größe nachzuweisen.

## 3. Klonierung des Gesamtfragments in pDH51

Das Fragment zur Expression der multifunktionellen RNA wurde durch SmaI Verdau aus der pBluescript Konstruktion isoliert und in einem SmaI geschnittenen pDH51 eingebaut. Die Orientierung des Fragments konnte durch Hind III bestimmt werden.

Das Plasmid pDH51 wird in Pietrzak, M. et al., NAR 14 , 5857-5869 (1986) nacharbeitbar beschrieben.

Das Bild zeigt DNA-Minipräparationen von MC1061 Zellen nach Transformation mit pDH51, in welches das multifunktionelle DNA-Fragment in die SmaI Stelle eingebaut wurde.

Die DNA-Minipräparationen wurde Hind III gespalten. Ein Einbau mit der gewünschten Orientierung erscheint in den Präparationen 1, 3, 5, 8 und 9.

EP 0 421 376 B1

### 4. Klonierung Gesamtfragment mit 355 Promotor in pOCA18

Das 1,2 kbp Fragment zur Expression der mulifunktionellen RNA wurde zusammen mit dem 355 Promotor und Terminator durch einen Eco RI-Verdau des pDH51 mit dem klonierten Insert isoliert. Das isolierte Fragment ligierte man mit einem Eco RI geschnittenen pOCA18 Vektor. Der mit dem Fragment ligierte Vektor wurde in MC1061 Zellen durch Transformation überführt.

Das Plasmid pOCA18 wird in Olszewski, N. et al. NAR 16, 10765-10782 (1988) nacharbeitbar beschrieben.

Das Bild zeigt DNA Minipräparationen aus MC1061 Zellen, die mit pOCA18, in den das Eco RI Fragment ligiert wurde, transformiert waren. Die DNA wurde vor dem Auftragen auf das Gel mit Eco RI gespalten (mit " + " bezeichnet). Daneben wurde jedesmal dieselbe Menge ungespaltene DNA aufgetragen ("-"). Die Kolonie 5 aus diesem Versuch ergab eine Eco RIBande der gewünschten Größe.

### 5. Transformation von Agrobakterien

Der pOCA18 Vektor mit dem 35S Promotor/Oligonukleotid-Insert wurde in den Agrobakterienstamm A 282 (Pharmacia Freiburg, BR Deutschland, oder ATCC 37349, USA) überführt. Dies geschah durch ein Triparental Mating mit Hilfe des E. coli Stammes SM10 (Simon, R. et al. Bio/Technology 1, 784-791, 1983). Dazu gab man gleiche Mengen der Bakterien gemeinsam über Nacht auf einen Filter, spülte mit 2 ml 10 mM $MgSO_4$ ab und gab Aliquote davon auf YEB-Platten mit Tetrazyklin und Rifampicin (YEB: 1% Hefeextrakt, 1% Pepton, 0,5% NaCl). Positive Agrobakterien konnten durch Hybridisierung nachgewiesen werden. Dazu überführt man die Kolonien auf GeneScreen Plusfilter (New England Nuclear, Boston). Die Filter wurden auf YEB Platten über Nacht bei 28°C inkubiert und am nächsten Tag denaturiert und neutralisiert. Die Filter hybridisierte man anschließend mit $4\times10^5$ cpm/nl des radioaktiv markierten Gesamtfragments bei 65°C über Nacht.

Der Filter wurde 1 x mit 1xSSC und 2 x mit 0,1xSSC/ SDS jeweils 30 Minuten bei 65°C gewaschen.

Das Bild zeigt einen Film, der 6 Stunden auf den gewaschenen Filtern exponiert wurde. Die Kolonie 3 ist positiv. Diese wurde zweifach aufgetragen, um das Ergebnis aus einem vorhergehenden Versuch zu bestätigen.

## 6. Transformation von Tabak

Die Agrobakterien wurden in YEB Medium (1% Hefeextrakt, 1% Pepton, 0,5% NaCl) mit Tetrazyklin und Rifampicin angezüchtet, 20 ml der Bakterien wurden abzentrifugiert, einmal in YEB Medium gewaschen und in 20 ml 10 mM $MgSO_4$ suspendiert in eine Petrischale gegeben. Als Pflanzenmaterial wurde Nicotiana tabacum, Wisconsin 38 verwendet. Die Pflanzen waren 4 Wochen unter sterilen Bedingungen auf 2MS Medium (Murashige T. et al., Physiol. Plant 15, 473-497 (1962)) bei 25°C mit 16 Std. Licht pro Tag kultiviert worden. Von diesen Pflanzen wurde ein 1 $cm^2$ Blattstückchen abgeschnitten, mit sterilem Schmirgelpapier verwundet und 30 sec in die Bakterienkultur eingetaucht. Die Blattstückchen wurden 2 Tage bei 25°C auf MS Medium, wie oben für 2MS beschrieben, gehalten und anschließend mit flüssigem 2MS Medium gewaschen. Danach kamen die Blattstückchen auf MSC 10 (wie MS mit 1,5% Agar) Platten mit Kanamycin. Nach 5-6 Wochen konnten regenerierte Pflanzen in größere Gefäße umgepflanzt werden, wo sie nach 2-3 Wochen Wurzeln bildeten.

Aus transgenen Tabakpflanzen wurde nach der CTAB-Methode DNA isoliert. Man hielt sich dabei genau an das Protokoll des "Plant Molecular Biology Manual", KLuwer Academic Publishers, Dordrecht (1988). 10 $\mu$g der DNA wurden E RI gespalten, auf einem 1% Agaoxgel aufgetrennt und nach Denaturieren auf eine Gene Screen Plus Membran transferiert. Die Membran wurde mit $4 \times 10^5$ cm/ml des radioaktiv markierten Fragments über Nacht bei 65°C hybridisiert. Ein Fragment der erwarteten Größe konnte in allen Fällen nachgewiesen werden.

```
Bild:   10 µg Gesamt DNA mit Eco RI verdaut hybridisiert
        mit Gesamtfragment
```

```
1) λ PST           7) A-H6
2) Kontrolle       8) A-H7
3) A-H1            9) A-H9
4) A-H3           10) A-H10
5) A-H4           11) A-H12
6) A-H5           12) A-H15
                  13) A-H22
```

Alle DNA-Präparationen aus transgenen Pflanzen zeigen die erwartete Hybridisierung mit einer 1,2 kb Eco RI Bande.

**7. Expression der multifunktionellen RNA mit transgenen Tabakpflanzen**

Aus Blattstücken transgener Tabakpflanzen wurde Gesamt-RNA isoliert. Man hielt sich dabei an das Protokoll des "Plant Molecular Biology Manual", Kluwer Academic Publishers, Dordrecht (1988). 10 µg der Gesamt-RNA wurden nach Denaturierung auf einem 2,2 M Formaldehyd/1% Agarosegel in 12mM Tris pH 7,5/0,1 mM EDTA-Puffer aufgetrennt und auf eine Gene Screen Plus Membran übertragen. Die Membran wurde mit radioaktiv markiertem Gesamtfragment hybridisiert. Die transgenen Pflanzen zeigen eine starke RNA-Expression mit der erwarteten Spezifität.

**8. Infektion transgener Tabakpflanzen mit CMV**

Die Infektion erfolgte mit dem CMV Stamm Q (Rezaian, M. et al., Eur. J. Biochem. 150, 331 (1985); 143, 277 (1984); Gould, J. et al., Eur. J. Biochem. 126, 217 (1982)), der aus Gurkenblättern gewonnen wurde. Die infektiösen Partikel wurden mit Hilfe von Carborundum auf die Tabakpflanzen aufgebracht. Die Kontrollpflanzen zeigten bei den gewählten Infektionsbedingungen zu 80% starke Symptome, die aus Adernaufhellungen, Mosaikbildungen und unregelmäßigen Wachstum der Blattränder bestanden. Die Spezifität der Infektion konnte durch ELISA-Test bestätigt werden.

Der Test der Transgenität wurde an Pflanzen durchgeführt, die vorher durch Stecklinge vermehrt worden waren. Eine Bestätigung an den Nachkommen wurde vorgenommen, sofern Samen vorlagen.

Es zeigte sich, daß praktisch in allen Pflanzen, die eine Expression der multifunktionellen RNA zeigten, eine starke Reduzierung der Infektionswahrscheinlichkeit eingetreten war. Nur etwa 10-50% der transgenen Pflanzen, abhängig jeweils von einem spezifischen Transformationsereignis, bildeten die typischen Infektionssymptome aus.

Tabelle

| Prozentwerte infizierte Pflanzen mit deutlichen Symptomen; je 10 Pflanzen wurden ausgewertet | | | |
|---|---|---|---|
| | | 12 Tage | 20 Tage |
| Kontrolle Tabak W38 | | 80 | 80 |
| Transgene: | A-H3 | 20 | 30 |
| | A-H7 | 10 | 20 |
| | A-H15 | 40 | 50 |
| | A-H22 | 30 | 30 |

Die verwendete Methode erwies sich somit als geeignet, Pflanzen vor Virusinfektionen zu schützen. Zur weiteren Verbesserung der Resistenz kann versucht werden, noch mehr Elemente an die multifunktionelle RNA anzuhängen.

Sequenz zur Expression einer multifunktionellen RNA

Tabelle 1

```
XmaI
                9               18              27              36
5' CCG GGA GGT AGC TCC TGA TGA GTC CGT GAG GAC GAA ACA ACC
3'   C CCT CCA TCG AGG ACT ACT CAG GCA CTC CTG CTT TGT TGG

      45              54              63              72              81
      TTG TCG TCG ACA AAA TGG TCA GTA TGC CCC TCG AGT GGT CTC
      ACC AGC AGC TGT TTT ACC AGT CAT ACG GGG AGC TCA CCA GAG

              90              99              108             117 KpnI   126
          CTT ATG GAG AAC CTG TGG AAA ACC ACA GGC GGT ACC CGC ACT
          GAA TAC CTC TTG GAC ACC TTT TGG TGT CCG CCA TGG GCG TGA

              135  .          144             153             162
          CTT GGT AAT ATC AGT GTA TTA CCG TGC ACG AGC TTC TCA CGA
          GAA CCA TTA TAG TCA CAT AAT GGC ACG TGC TCG AAG AGT GCT

      171             180             189             198             207
      AGC CCT TCC GAA GAA ATC TAG GAG ATG ATT TCA AGG GTA GCT
      TCG GGA AGG CTT CTT TAG ATC CTC TAC TAA AGT TCC CAT CGA

              216     BamHI 225             234             243             252
          CGA CAA CCT GGA TCC AAA ATG GTC AGT ATG CCC CCC ATG GCA
          GCT GTT GGA CCT AGG TTT TAC CAG TCA TAC GGG GGG TAC CGT

              261             270             279             288
          ACA GAT TGG CGA ATG AGA AAG TGG GTG GAG GAC TTA TCA TAG
          TGT CTA ACC GCT TAC TCT TTC ACC CAC CTC CTG AAT AGT ATC

      297             306             315             324             333
      TAA CAG AAG AGA GAC TAG AAC TGC AGA AAA TGG TCA GTA TGC
      ATT GTC TTC TCT CTG ATC TTG ACG TCT TTT ACC AGT CAT ACG

              342             351             360             369     SacI 378
          CCC AGA TCT ACC GGA GGT TCT ACT AGC ATT GGG AGA GCT CGA
          GGG TCT AGA TGG CCT CCA AGA TGA TCG TAA CCC TCT CGA GTC

              387             396             405             414
          TTT GTC CAT AGG CAC ACT GAG ACG CAA AAA GCT TAA GGT TGT
          AAA CAG GTA TCC GTG TGA CTC TGC GTT TTT CGA ATT CCA ACA

      423             432             441             450             459
      CGA GCT ACC GGG GCC CAG GGC ATA CTC TGA TGA GTC CGT GAG
      GCT CGA TGG CCC CGG GTC CCG TAT GAG ACT ACT CAG GCA CTC

              468             477 Sma
          GAC GAA ACC ATT TTG GG 3'
          CTG CTT TGG TAA AAC CC 5'
```

**Patentansprüche**

1. Gen, das für eine multifunktionelle RNA kodiert, die in 5'-3'-Richtung der Transcription umfaßt:

   a, Ribozym-RNA

   b, Spacer

   c, (Antisense-RNA)$_n$-,

   d, Spacer und

   e, Ribozym-RNA

   wobei

   - n eine Zahl von 0 bis 10 ist,
   - als Spacer eine Kette von 20 bis 25 Nukleotiden eingeschoben wird, die von dem Ribozym geschnitten werden kann und
   - die Ribozyme die folgende Struktur aufweisen:

   worin

   N     Nukleotide der Substrat-RNA, A, C, G oder U,

   K     komplementäre Nukleotide zu N im Ribozym,

   V     variable Nukleotide A, C, G oder U im Ribozym

   $V_L$     0-550 variable Nukleotide A, C, G oder U im Loop des Ribozyms bedeutet.

2. Gen nach Anspruch 1 oder 2, dadurch gekennzeichnet, daß die kodierte Antisense-RNA zu einer Virus-RNA komplementär ist.

3. Gen nach einem oder mehreren der Ansprüche 1 bis 3, dadurch gekennzeichnet, daß die kodierte Ribozym-RNA zu einer Virus-RNA komplementär ist.

4. Gen nach einem oder mehreren der Ansprüche 1 bis 4, gekennzeichnet durch die in Tabelle 1 aufgeführte DNA-Sequenz.

5. Multifunktionelle RNA kodiert durch ein Gen nach einem der Ansprüche 1 bis 5.

6. Wirtszelle, enthaltend ein Gen nach einem der Ansprüche 1 bis 4.

7. Wirtszelle, enthaltend eine RNA nach Anspruch 5.

8. Pflanzen, Pflanzenteile sowie Teile oder Samen der Pflanzen, dadurch gekennzeichnet, daß sie das Gen nach einem der Ansprüche 1 bis 4 enthalten.

9. Pflanzen, Pflanzenteile sowie Teile oder Samen der Pflanzen, dadurch gekennzeichnet, daß sie eine RNA nach Anspruch 5 enthalten.

10. Verwendung der Gene gemäß mindestens einem der Ansprüche 1 bis 4 kodierten RNA als antivirales Agens in Pflanzen.

**Claims**

1.  A gene which codes for a multifunctional RNA which comprises in the 5'-3' direction of transcription:
    a, ribozyme RNA
    b, spacer
    c, (antisense RNA)$_n$-,
    d, spacer and
    e, ribozyme RNA
    where
    - n is a number from 0 to 10,
    - a chain of 20 to 25 nucleotides which can be cut by the ribozyme is inserted as spacer and
    - the ribozymes have the following structure:

    in which
    N       are nucleotides of the substrate RNA, A, C, G or U,
    K       are nucleotides complementary to N in the ribozyme,
    V       are variable nucleotides A, C, G, or U in the ribozyme,
    V$_L$    are 0-550 variable nucleotides A, C, G or U in  the loop of the ribozyme.

2.  The gene as claimed in claim 1, in which the encoded antisense RNA is complementary to a viral RNA.

3.  The gene as claimed in either of claims 1 or 2, in which the encoded ribozyme RNA is complementary to a viral RNA.

4.  The gene as claimed in one or more of claims 1 to 3, which comprises the DNA sequence listed in Table 1.

5.  A multifunctional RNA encoded by a gene as claimed in one of claims 1 to 4.

6.  A host cell containing a gene as claimed in one of claims 1 to 4.

7.  A host cell containing an RNA as claimed in claim 5.

8.  Plants, plant cells and parts or seeds of plants, which contain the gene as claimed in one of claims 1 to 4.

9.  Plants, plant cells and parts or seeds of plants, which contain an RNA as claimed in claim 5.

10. The use of the RNA encoded by the genes as claimed in at least one of claims 1 to 4 as antiviral agent in plants.

**Revendications**

1.  Gène codant pour un ARN multifonctionnel qui comprend, dans le sens 5'-3' de la transcription:

a) l'ARN d'un ribozyme

b) un espaceur

c) (ARN antisens)$_n$,

d) un espaceur et

e) l'ARN d'un ribozyme

dans lequel

- n est un nombre de 0 à 10

- on intercale comme espaceur une chaîne de 20 à 25 nucléotides qui peut être coupée par le ribozyme, et

- les ribozymes ont la structure suivante:

```
5-NNNNNNNNNNNNGUC NNNNNNNNNNNNNN-3
3-KKKKKKKKCA  KKKKKKK-5
              A   C
              A    U
              A     G
              G   A   A
              C G  G V
              V V
              V V
              V V V
              V V
```

dans laquelle

les N représentent des nucléotides de l'ARN du substrat, A, C, G ou U,

les K sont des nucléotides complémentaires de N dans le ribozyme,

les V représentent des nucléotides variables A, C, G ou U dans le ribozyme,

les $V_L$ représentent 0 - 550 nucléotides A, C, G ou U dans la boucle du ribozyme.

2. Gène selon la revendication 1, caractérisé en ce que l'ARN antisens pour lequel il code est complémentaire d'un ARN viral.

3. Gène selon la revendication 1 ou 2, caractérisé en ce que l'ARN de ribozyme pour lequel il code est complémentaire à un ARN viral.

4. Gène selon l'une ou plusieurs des revendications 1 à 3, caractérisé par la séquence d'ADN présentée dans le tableau 1.

5. ARN multifonctionnel pour lequel code un gène selon l'une des revendications 1 à 4.

6. Cellules hôtes, contenant un gène selon l'une des revendications 1 à 4.

7. Cellules hôtes, contenant un ARN selon la revendication 5.

8. Plantes, parties de plantes, et parties ou graines des plantes, caractérisées en ce qu'elles contiennent le gène selon l'une des revendications 1 à 4.

9. Plantes, parties de plantes, et parties ou graines des plantes, caractérisées en ce qu'elles contiennent un ARN selon la revendication 5.

10. Utilisation de l'ARN pour lequel code un gène selon l'une au moins des revendications 1 à 4 comme agent antiviral chez les plantes.